# EUROPEAN PATENT SPECIFICATION

(11) **EP 1 144 018 B1**
(45) Date of publication and mention of the grant of the patent: **24.03.2004**
(21) Application number: 99966346.1
(22) Date of filing: 16.12.1999
(51) Int. Cl.: A61L 27/56, A61L 27/42, A61L 27/46, A61L 24/04, A61P 19/00

(54) **SEQUENCED INCORPORATION OF CORTICAL BONE ALLOGRAFTS**
SEQUENZIERTE INKORPORATION VON KORTIKALKNOCHENTRANSPLANTATEN
INCORPORATION SEQUENTIELLE D'ALLOGREFFES DE PERIOSTE

(30) Priority: 17.12.1998 US 112596 P
(43) Date of publication of application: 17.10.2001
(73) Proprietor: CAMBRIDGE SCIENTIFIC, INC., Cambridge, MA 02138 (US)
(72) Inventor: LEWANDROWSKI, Kai-Uwe, Brookline, MA 02176 (US); TRANTOLO, Debra, J., Princeton, MA 01541-1806 (US)
(74) Representative: Bassett, Richard Simon
(86) International application number: PCT/US1999/029981
(87) International publication number: WO 2000/035511

(56) References cited:
- WO-A-98/22041
- WO-A-99/11789
- WO-A-99/32166
- US-A- 5 766 618
- US-A- 5 837 752
- MASON J M ET AL: "Expression of human bone morphogenic protein 7 in primary rabbit periosteal cells: Potential utility in gene therapy for osteochondral repair" GENE THERAPY,GB,MACMILLAN PRESS LTD., BASINGSTOKE, vol. 5, no. 8, 1 August 1998 (1998-08-01), pages 1098-1104, XP002087788 ISSN: 0969-7128

## Description

### Field of the Invention

Materials and methods for use thereof for producing a three-dimensional matrix in bone having a physical appearance and mechanical stability similar to trabecular bone, using a biodegradable, biocompatible polymer, preferably in conjunction with an osteoconductive, osteoinductive and/or buffering filler, are disclosed.

### Background of the Invention

Trauma, pathological degeneration, or congenital deformity of tissues may result in the need for surgical reconstruction or replacement. Reconstructive surgery is based upon the principle of replacing these defective tissues with viable. functioning alternatives. Surgeons have historically used bone grafts to correct skeletal defects. The two main types of bone grafts currently used are autografts and allografts. An autograft is a section of bone taken from the patient's own body, while an allograft is taken from a cadaver. This method of grafting provides the defect site with structural stability and natural osteogenic behavior. However, both types of grafts are limited by certain uncontrollable factors. For autografts, the key limitation is donor site morbidity where the remaining tissue at the harvest site is damaged by removal of the graft. Other considerations include the limited amount of bone available for harvesting, and unpredictable resorption characteristics of the graft. Large frozen cortical bone allografts have been increasingly used in limb sparing procedures not only in the treatment of bone tumors, after massive bone loss following traumatic injuries, or in the treatment of avascular necrosis, but also in failed joint arthroplasties, where extensive bone loss due to osteolysis is commonly encountered.

Human bone obtained from cadaveric donors is typically procured under sterile conditions in an operating room environment at a local hospital. The bone is stored frozen until it is further processed into small grafts under sterile or clean-room conditions. Procurement and processing of human tissues is typically performed by groups certified by the American Association of Tissue Banks under standard operating procedures for the processing of each specific bone graft. Large bones such as the femur are thawed and debrided of excess tissue prior to being cut into smaller grafts. Processing of the smaller grafts includes cleaning of bone marrow from the cancellous bone spaces. Cleaning of bone marrow from small bone grafts has been described in the scientific literature and in brochures and documents made public by groups involved in the procurement and processing of human tissues. These processes remove bone marrow. tissue, lipids and blood components, using a variety of materials including solvents and surfactants.

These types of grafts are used in the management of large skeletal defects which continue to present a challenge to orthopedic surgeons, particularly when the problem arises in young patients in whom artificial devices and joint implants are likely to lead to early failure. The main limitation of allografts has been the immunologic response to the foreign tissue of the graft. The tissue is subject to the inflammatory response and may be rejected. Allografts are also capable of transmitting disease. Although a thorough screening process eliminates most of the disease carrying tissue, this method is not completely effective.

Although the overall success rate, implying return to work and engagement in relatively normal activities without crutches or braces, is approximately 75-85%, only 50% of these patients have an entirely uncomplicated postoperative course. About a quarter of the total group require repeat operations such as autologous grafting or replating for stress fractures delayed unions. Some patients require excision of the graft because of infection, reimplantation, long-term bracing or in some cases amputation. These results clearly indicate that problems still exist with this procedure and that if the technique is to be more widely applied, it must be more extensively examined and materially improved.

Clinical and experimental reports provide sufficient evidence to conclude that massive cortical bone allografts serve primarily as osteoconductive scaffolds with poor osteoinductive potential. In fact. new bone is often found to penetrate into the graft only a few millimeters from its peripheral surfaces. Resorption of the non-viable bone of allogenic cadaveric bone implants often forms the basis for clinical complications. Thus, better control of bone resorption and stimulation of new bone formation may ultimately improve clinical outcomes.

Previous studies have attempted to improve host incorporation by altering the geometrical surface configuration of cortical bone (Bernick, 1989; Gendler, 1986; 1990; O'Donnell, 1996; Scanlon, 1991; Sires, 1992). The mechanism by which the presence of perforations promotes osteogenesis and incorporation in demineralized grafts may be a function of either the greater surface area of partially demineralized bone or increased access to vascular tissue, or both. Previous studies have pointed out that the geometry of an implant may influence the extent of bone formation. For example, the osteoconductive potential of coralline hydroxyapatite ("HA") with a pore size of 0.6 mm has been attributed, at least in part, to its morphometric similarity to cancellous bone (Holmes, 1986). As such, the osteoconductive potential of cortical bone allografts treated to yield likewise structures may be similar. Gendler used fully demineralized diaphyseal allogeneic struts that were perforated with the use of a mechanical drill (1986). In comparison. O'Donnell et al. used demineralized calvarial cortical bone (1996). Bernick et al. (1989) characterized the inductive cellular events in a similar system. Scanlon implanted demineralized canine femoral strut allografts in an orthotopic model (1991). The later two studies have demonstrated the use of an Erbium: Yttrium-Scandium-Gallium-Garnet (Er:YSGG) laser for drilling of cortical bone allografts, thereby increasing the porosity and allowing demineralization to proceed to areas that would normally be inaccessible to the demineralization process. When reimplanted, these grafts appear to be more osteogenic than cortical grafts without holes.

Although demineralization has been successful in improving osteoinductive properties of bone, as shown by the abundance of pertinent literature on experimental and clinical studies, fully demineralized cortical bone has found limited clinical application. This appears primarily attributable to the fact that cortical bone, once subjected to extensive demineralization, has lost its essential biomechanical properties.

Defects in the process of bone repair and regeneration are linked to the development of several human diseases and disorders, e.g., osteoporosis and osteogenesis imperfecta. Failure of the bone repair mechanism is, of course, also associated with significant complications in clinical orthopedic practice, for example. fibrous non-union following bone fracture. implant interface failures and large allograft failures. The lives of many individuals would be improved by the development of new types of bone grafts designed to stimulate and strengthen the healing and repair process.

Conventional orthopedic implants such as screws, plates, pins and rods serve as load-bearing replacements for damaged bone and are usually composed of a metal or alloy. Although these implants are capable of providing rigid fixation and stabilization of the bone, they cause improper bone remodeling of the implant site due to the large difference in the modulus between bone and metal.

The problems with these materials have led to research for dependable synthetic adjuncts to bone grafts and bone graft substitutes. However, in order for an implant to be used as a replacement for bone, it must be capable of both osteointegration and osteoconduction. Osteointegration refers to direct chemical bonding of a biomaterial to the surface of bone without an intervening layer of fibrous tissue. This bonding is referred to as the implant-bone interface. A primary problem with skeletal implants is mobility. Motion of the implant not only limits its function, but also predisposes the implant site to infection and bone resorption. With a strong implant-bone interface, however, mobility is eliminated, thus allowing for proper healing to occur. Osteoconduction refers to the ability of a biomaterial to sustain cell growth and proliferation over its surface while maintaining the cellular phenotype. For osteoblasts, the phenotype includes mineralization, collagen production, and protein synthesis. Normal osteoblast function is particularly important for porous implants that require bone ingrowth for proper strength and adequate surface area for bone bonding.

Calcium phosphate-based materials have been widely investigated for use as bone replacement materials. Most calcium phosphate biomaterials are polycrystalline ceramics characterized by a high biocompatibility, the ability to undergo osteointegration, and varying degrees of resorbability. Implants made from these materials can be in either a porous or non-porous form. Examples of commercially available calcium phosphate materials include Interpore™ 2000 and Interpore™ 5000. Surgical models using porous calcium phosphate-based implant materials, however, have shown that porous implants heal more slowly than both autografts and empty defects.

Any new technique to stimulate bone repair would be a valuable tool in treating large bony defects. A very significant patient population that would benefit from new therapies designed to promote bone allograft incorporation, or even prevent or lessen the incidence of fractures, and infections, are those patients suffering from large defects of their skeletal system. An estimated 20-25 million people are at increased risk for fracture because of site-specific bone loss. The cost of treating osteoporosis in the United States is currently estimated to be in the order of $10 billion per year. Demographic trends, i.e., the gradually increasing age of the US population, suggest that these costs may increase 2-3 fold by the year 2020 if a safe and effective treatment is not found.

It is therefore an object of the present invention to provide allograft and implant materials that are more biocompatible, and promote better osteointegration and osteoconduction.

### Summary of the Invention

Methods and materials for filling perforations in partially demineralized cortical bone allografts with matrices including a biodegradable, biocompatible polymer, preferably in conjunction with osteoconductive, osteoinductive, filler or bulking agent and/or buffering material, such as HA, are disclosed. The matrix enhanced control of resorption and bony ingrowth in the cortical bone allografts. The matrix can be used to deliver factors to bone progenitor cells *in vivo* to stimulate bone. The matrix can also be used to transfer the stimulatory factor into the cells of the soft tissues surrounding the cortical bone allograft or cultured cells or recombinant cells maintained *in vitro,* where all that is required is to add the stimulatory factor composition to the cells, e.g., by adding it to the culture media. The matrix may be employed to promote expression of a desired gene in bone cells or tissues surrounding the cortical bone allograft and to impart a particular desired phenotype to the cells. This expression could be increased expression of a gene that is normally expressed (i.e., "over-expression"), or it could be used to express a gene that is not normally associated with bone progenitor cells in their natural environment. Alternatively, the matrix may be used to suppress the expression of a gene that is naturally expressed in such cells and tissues, and again, to change or alter the phenotype. Gene suppression may by expression of a gene that encodes a protein that exerts a down-regulatory function, or it may utilize antisense technology.

### Detailed Description of the Invention

The process of bone repair and regeneration resembles the process of wound healing in other tissues. A typical sequence of events includes hemorrhage; clot formation; dissolution of the clot with concurrent removal of damaged tissues; ingrowth of granulation tissue; formation of cartilage; capillary ingrowth and cartilage turnover; rapid bone formation (callus tissue); and, finally, remodeling of the callus into cortical and trabecular bone. Bone repair is a complex process that involves many cell types and regulatory molecules. The diverse cell populations involved in fracture repair include stem cells, macrophages, fibroblasts. vascular cells, osteoblasts. chondroblasts, and osteoclasts.

As described herein, polymer-allografts having improved properties are formed by coating and/or injecting polymer alone or in combination with bioactive materials into the bone material, preferably after the porosity of the bone has been increased or the surface texture altered. In the preferred embodiment. the polymeric material will include osteotropic factors associated with, or impregnated within. This polymeric composite is referred to as a bone compatible matrix, and is used to form a "matrix-bone allograft composition". The matrix-bone allograft composition is then placed in contact with the bone progenitor cells or tissue, prior to or at the time of implantation.

### 1. Materials for forming Polymer-Allograft Composites

### Biodegradable Polymeric Materials and Bone-Compatible Matrices

The principal component of the matrix is one or more polymers, copolymers or blends of polymers, which can then include osteoinductive, osteoconductive, fillers, and/or buffering agents. Polymers are a class of synthetic and naturally occurring materials characterized by their high versatility. The choice of polymeric material will differ according to the particular circumstances and the site of the bone that is to be treated. Physical and chemical characteristics, such as, e.g., biocompatibility, biodegradability, strength, rigidity, interface properties and even cosmetic appearance are factors that may be considered in selecting a polymeric.

A number of biodegradable, biocompatible polymers have been created primarily for use in medical applications and are preferred for use herein. As used herein, the term "biocompatible" means that the material does not produce an adverse, allergic or other unacceptable reaction when implanted in an animal, and is compatible with bone tissue. The term "biodegradable" refers to the material breaking down into its basic components over a period of time following induction into the body, typically by hydrolysis and/or enzymolysis, usually over a period of less than two years, more typically less than one year.

One of the most common polymers used as a biomaterial is the polyester copolymer poly(lactic acid-glycolic acid) (PLGA). PLGA is highly biocompatible, degrades into biocompatible monomers and has a wide range of mechanical properties making this copolymer and its homopolymers, PLA and PGA, useful in skeletal repair and regeneration. Porous, three-dimensional matrices comprising these polymers for use in bone replacement have been prepared using various techniques. A biodegradable. biocompatible polymer foam composed of a poly(lactic acid-co-glycolic acid) [PLGA], preferably prepared as a composite with a calcium phosphate ceramic, such as HA, is a preferred material for engineering of surface modifications of cortical bone allografts. In conjunction with an osteoconductive buffering agent such as HA. these porous polymeric matrices are particularly useful as cellular scaffolds for bone regeneration. Matrices such as those described in U.S. Patent No. 5,270,300 may also be employed.

These materials can be used to modify cortical bone allografts to yield a bone reconstruction material that can precisely fit a defect site, while temporarily serving as a template for new bone generation. It provides a reinforcing structure to a scaffold for new bone growth. The concept of porosity control is based on the use of degradable polymers in conjunction with porous cortical allograft. Chemical hydrolysis of the hydrolytically unstable backbone of the PLGA polymers is the prevailing mechanism for polymer degradation. This occurs in two phases. In the first phase, water penetrates the bulk of the polymer-filled pores, preferentially attacking the chemical bonds in the amorphous phase and converting long polymer chains into shorter water-soluble fragments. Because this occurs initially in the amorphous phase, there is a reduction in molecular weight without a loss in physical properties since the polymer matrix is still held together by the crystalline regions. The reduction in molecular weight is soon followed by a reduction in physical properties, as water begins to fragment the material. In the second phase, enzymatic attack and metabolization of the fragments occurs, resulting in a rapid loss of polymer mass. This type of degradation, when the rate at which water penetrates the material exceeds that at which the polymer is converted into water-soluble materials (resulting in erosion throughout the matrix), is termed "bulk erosion" (Hubbell and Langer, 1995). The rate of degradation of PLGA's can be controlled, in part. by the copolymer ratio with higher glycolide or lactide ratios favoring longer degradation times. Polymers of varying copolymer ratios including PLA, PLGA75:25, and PLGA50:50 were compared in the following example to examine the effect of degradation rates.

Other polymeric materials that can be used include synthetic and natural polymers, including other polyesters, polyanhydrides, polyhydroxyalkanoates, poly(glaxanone), poly(orthoesters), and poly(phosphazenes), ethylenevinylacetate, polycarbonates, proteins and polysaccharide materials. Suitable acrylic ester polymers and lactic acid polymers are disclosed in U.S. Patent Nos. 4,526, 909, and 4,563,489. Other biodegradable materials include, for example matrices of purified proteins, and semi-purified extracellular matrix compositions. Preferred matrix materials are those prepared from tendon or dermal collagen, available from a variety of commercial sources, such as, e.g., Sigma and Collagen Corporation. Collagen matrices may also be prepared as described in U.S. Patent Nos. 4,394,370 and 4,975,527. Currently, the most preferred collagenous material is UltraFiber™, obtainable from Norian Corp. (Mountain View, Calif.).

In certain embodiments, non-biodegradable matrices may be employed, such as sintered HA, bioglass, aluminates, other bioceramic materials and metal materials, particularly titanium. A suitable ceramic material is described in U.S. Patent No. 4,596,574. Biodegradable and chemically defined calcium sulfate, tricalcium phosphate, and HA can also be used. Implants synthesized from the calcium phosphate-based material, HA, the major mineral constituent of bone, are commercially available in a porous and non-porous form. Synthetic HA implants have excellent biocompatibility. Blocks of dense HA are not useful in reconstructive surgery because they are difficult to shape and do not permit tissue ingrowth. However, in a non-porous, particulate form, HA has been used successfully in both composite (Collagraft™) and cement (Hapset™) forms (Chow et al. Mater. Res. Soc. Symp. Proc. 1993 179:3-24; Cornell, C. N. Tech. Orthop. 1992 7:55). Due to its fragility and lack of compliance, porous HA has been largely limited to use in dental and maxillofacial surgery.

Tricalcium phosphate (TCP) is the other main type of calcium-phosphate based implant material. It has a biocompatibility similar to HA, but it is more resorbable than HA due to its crystal structure. The chemical structure of TCP allows it to be used as a calcium phosphate cement (CPC) (Chow, L. C. Centen. Mem. Issue Ceramics Soc. Jpn 1991 99:954; Mirtchi et al. Biomaterials 1989 10:475) which can be mixed in the operating room and thus can be easily molded to fit the implantation site. The compliance of TCP materials allow them to be used in a broader range of surgical applications than conventional ceramics.

Other types of ceramic bone replacement materials are based on silicate. The use of silicate based materials in bone replacement is associated with its biocompatibility. Unlike calcium-phosphate materials, silicate does not exist naturally in the body. However, its biocompatibility is similar to naturally occurring minerals. Examples of silicate based bone replacements include bioactive glasses.

In some cases, these materials may have osteoinductive or osteoconductive properties, as discussed in more detail below. These materials can also be used as fillers or bulking agents, or buffering compounds. HA is a preferred buffering compound since it neutralizes acidic breakdown products of biodegradable polymers such as lactic acid and glycolic acid containing polymers, thereby diminishing the likelihood these materials could cause cytotoxicity, separation of the implant and sepsis.

### Bone Progenitor Cells and Tissues

The matrix can be used as a delivery agent for molecules which stimulate bone ingrowth (i.e., osteoconductive agents) and/or bone cell recruitment (i.e.. osteoinductive agents), or as a scaffolding for bone progenitor cells that are seeded onto or into the matrix, prior to or at the time of implantation, which may themselves produce osteoconductive and/or osteoinductive agents.

As used herein, the term "bone progenitor cells" refers to cells that have the capacity to ultimately form, or contribute to the formation of, new bone tissue. This includes various cells in different stages of differentiation, such as, for example, stem cells, macrophages, fibroblasts, vascular cells, osteoblasts, chondroblasts, and osteoclasts. Bone progenitor cells also include cells that have been isolated and manipulated *in vitro*, e.g., subjected to stimulation with agents such as cytokines or growth factors or even genetically engineered cells. The particular type or types of bone progenitor cells that are stimulated are not important, so long as the cells are stimulated in such a way that they are activated and, in the context of *in vivo* embodiments, ultimately give rise to new bone tissue.

The term "bone progenitor cell" is also used to particularly refer to those cells that are located within, are in contact with, or migrate towards (i.e., "home to"), bone progenitor tissue and which cells directly or indirectly stimulate the formation of mature bone. As such, the progenitor cells may be cells that ultimately differentiate into mature bone cells themselves, i.e., cells that "directly" form new bone tissue. Cells that, upon stimulation, attract further progenitor cells or promote nearby cells to differentiate into bone-forming cells (e.g., into osteoblasts, osteocytes and/or osteoclasts) are also considered to be progenitor cells as their stimulation "indirectly" leads to bone repair or regeneration. Cells affecting bone formation indirectly may do so by the elaboration of various growth factors or cytokines, or by their physical interaction with other cell types.

In terms of bone progenitor cells, these may also be cells that are attracted or recruited to such an area. These may be cells that are present within an artificially created osteotomy site in an animal model. Bone progenitor cells may also be isolated from animal or human tissues and maintained in an *in vitro* environment. Suitable areas of the body from which to obtain bone progenitor cells are areas such as the bone tissue and fluid surrounding a fracture or other skeletal defect (whether or not this is an artificially created site), or indeed, from the bone marrow. Isolated cells may be stimulated and then be returned to an appropriate site in an animal where bone repair is to be stimulated. In such cases, the cells can be used as therapeutic agents. Such *ex vivo* protocols are well known to those of skill in the art.

### Biometric Bone Stimulatory Factors

A variety of biomimetic and/or biodegradable materials have been utilized as stimulators of new bone formation. A particularly important aspect of the matrix material, especially the polymeric material, is its use in connection with orthopedic cortical bone allograft implants and interfaces with artificial joints, including implants themselves and functional parts of an implant, such as, e.g., surgical screws and pins. In one preferred embodiment, the surfaces of the bone allograft and/or implant or a portion thereof, such as a titanium surface, will be coated with a material that has an affinity for a bone stimulatory factor, most preferably HA, and then the coated-metal will be further coated with a gene encoding a bone stimulatory factor or the bone stimulatory factor itself that one wishes to incorporate. The gene or protein can be absorbed to, incorporated into, or chemically coupled to the matrix material within the pores of the bone graft or on the surface of the implant, using methods known to those skilled in the art.

Examples of stimulatory molecules include regulatory factors involved in bone repair, such as hormones, cytokines, growth factors, and other molecules that regulate growth and differentiation; and osteoinductive agents such as bone morphogenetic or morphogenic proteins (BMPs). The latter are also referred to as osteogenic bone inductive proteins or osteogenic proteins (OPs). Several BMP (or OP) genes have now been cloned, and the common designations are BMP-1 through BMP-8. Although the BMP terminology is widely used, it may prove to be the case that there is an OP counterpart term for every individual BMP (Alper. 1994). BMPs 2-8 are generally thought to be osteogenic, although BMP-1 is a more generalized morphogen (Shimell et al., 1991). BMP-3 is also called osteogenin (Luyten et al., 1989) and BMP-7 is also called OP-1 (Ozkaynak et al., 1990). BMPs are related to, or part of, the transforming growth factor-beta (TGF-beta) superfamily, and both TGF beta 1 and TGF beta 2 also regulates osteoblast function (Seitz et al., 1992). Several BMP (or OP) nucleotide sequences and polypeptides have been described in U.S. patents, e.g.. U.S. Patent Nos. 4,795,804, 4,877,864, 4,968,590, and 5,108,753; including, specifically, BMP-1 disclosed in U.S. Patent No. 5,108,922; BMP-2A in U.S. Patent Nos. 5,166,058 and 5,013,649; BMP-2B disclosed in U.S. Patent No. 5,013,649; BMP-3 in U.S. Patent No. 5,116,738; BMP-5 in U.S. Patent No.. 5,106,748; BMP-6 in U.S. Patent No. 5,187,076; BMP-7 in U.S. Patent Nos. 5,108,753 and 5,141,905; and OP-1, COP-5 and COP-7 in U.S. Patent No. 5,011,691.

Other growth factors or hormones that have been reported to have the capacity to stimulate new bone formation include acidic fibroblast growth factor (Jingushi et al., 1990); estrogen (Boden et al., 1989); macrophage colony stimulating factor (Horowitz et al., 1989); and calcium regulatory agents such as parathyroid hormone (PTH) (Raisz & Kream, 1983). Several groups have investigated the possibility of using bone stimulating proteins and polypeptides, particularly recombinant BMPs, to influence bone repair in vivo. For example, recombinant BMP-2 has been employed to repair surgically created defects in the mandible of adult dogs (Toriumi et al., 1991), and high doses of this molecule have been shown to functionally repair segmental defects in rat femurs (Yasko et al., 1992). Chen and colleagues showed that a single application of 25-100 ng of recombinant TGF-beta 1 adjacent to cartilage induced endochondral bone formation in the rabbit ear full thickness skin wounds (Chen et al., 1991). It has also been reported that an application of TGF-beta 1 in a 3% methylcellulose gel was able to repair surgically induced large skull defects that otherwise heal by fibrous connective tissue and never form bone (Beck et al., 1991).

Osteotropic proteins include besides transforming growth factor, fibroblast growth factor, granulocyte/macrophage colony stimulating factor, epidermal growth factor, platelet derived growth factor, insulin-like growth factor, and leukemia inhibitory factor.

In one embodiment, the gene or other nucleotide molecule encoding the stimulatory factor is administered rather than the protein. For example, the nucleotide molecule may be DNA (double or single-stranded) or RNA (e.g.. mRNA, tRNA, rRNA), or it may be an antisense nucleic acid molecule, such as antisense RNA that may function to disrupt gene expression or growth factors themselves including TGF-beta 1 and 2, and IGF-1. The nucleic acid segments may be genomic sequences, including exons or introns alone or exons and introns, or coding cDNA regions, or in fact any construct that one desires to transfer to a bone progenitor cell or tissue. Suitable nucleic acid segments may also be in virtually any form, such as naked DNA or RNA, including linear nucleic acid molecules and plasmids, or as a functional insert within the genomes of various recombinant viruses, including viruses with DNA genomes and retroviruses.

### II. Methods for Making the Polymer-Allograft Composites or Coatings

A three-dimensional matrix having a physical appearance and mechanical stability similar to trabecular bone is formed using a biodegradable, biocompatible polymer, preferably in conjunction with an osteoconductive buffering filler such as a calcium phosphate based material to create a porous, osteoconductive structure through the packing of perforations in partially demineralized cortical bone allografts with the polymer. Calcium phosphates can be used to buffer degradation products of the polymeric components without local accumulation of acidic degradation byproducts such as lactic acid. Examples of polymers which can be used include poly(lactic acid-glycolic acid) [PLGA], poly(lactic acid) [PLA], poly(glycolic acid)[PGA], poly(glaxanone), poly(orthoesters), and poly(phosphazenes). Examples of calcium phosphate based materials which can be used include HA and TCP.

Bone graft coatings or fillings can be used on a large number of essentially intact, or perforated, bone grafts, including femur, femur head, distal end of femur, proximal end of femur, fibula, tibia, ilia, mandibular, humerus, radius, ulna, vertebrae, ribs, scapula, foot bones and hand bones, prior to subsequent processing into small specific cut-bone grafts and of being usable on small cut-bone grafts, including iliac crest wedges, Cloward dowels, ribs, cancellous cubes, fibular struts. The process involves cleaning of the bone graft with removal of bone marrow from the interstitial lumen and cancellous bone space of large bone grafts and coating or filling with a polymer through the cartilaginous ends of the bone and out through lumen and cancellous bone space, or within the cancellous bone space of the small cut grafts.

Representative methods include solvent evaporation. Polymer is dissolved in a solvent. Examples of organic solvents which can be used to dissolve the polymer are well known in the art and include glacial acetic acid, methylene chloride, chloroform, tetrahydrofuran, and acetone. Allograft bone samples, perforated and preferably demineralized, are immersed in the polymer solution. The polymer solution is forced into the perforations using continuous evacuation and repressurization, the endpoint determination of hole filling being the absence of bubbles emerging from the holes. A polymer foam microstructure is then created within the perforations by freezing the bone/polymer and lyophilizing the solvent.

Accurate control over pore size in the polymer foam correlates with a high degree of control over pore size in the reconstructed bone matrix. The desired pore size for a bone replacement matrix falls between 150-250 µm (Hulbert et al., J. Biomed. Mat. Res. 1970 4:443). Accordingly, control of the polymer foam pore size is a significant factor in the ability of matrices to be used successfully in bone replacement procedures.

In another embodiment of this filling of perforations, a polymer/ceramic matrix is prepared from a mixture of polymer solution and a calcium phosphate. Polymer solutions are mixed with the calcium phosphate based material. The bone is then immersed in this mixture and the composite frozen and lyophilized.

In another embodiment, a polymer solution is combined with a osteoinductive material with or without a buffering or osteoconductive filler. Polymer solutions are mixed with the osteoinductive agent with or without the filler such as the calcium phosphate based material. The bone is then immersed in this mixture and the composite frozen and lyophilized.

In still another embodiment, a solvent cast microspherical polymer is prepared. In this method, polymer microspheres are mixed with void forming particles and a calcium phosphate based material. An organic solvent capable of dissolving the polymer, such as methylene chloride, chloroform, tetrahydrofuran or acetone, is added to the mixture in a dropwise fashion with stirring so that the polymer microspheres of the mixture aggregate.

Alternatively, the implant material can be of a moldable, putty form that will eventually harden and be able to withstand normal physiological stresses. Because the material is in putty form, implants of this nature are usually less porous. Conventional materials of this type have been characterized as bone cements. Although bone cements are in a self-setting putty form and have the required mechanical properties, they are generally not resorbable and eventually fail after prolonged implantation. By using biodegradable putty materials, the dynamics of bone resorption and new bone formation following bone allograft transplantation can be modulated and controlled and, hence, avoid problems associated with their implantation. This step-wise degradation of the matrix within the perforations of partially demineralized cortical bone allografts provides the implant site with decreasing reinforcement which allows for the gradual ingrowth of newly formed bone. Since bone is a dynamic tissue that responds to changes in stress, gradual loading of the regenerating bone stimulates further bone formation without causing stress damage to the cortical bone allograft. Thus, the composite of cortical bone allograft and osteoinductive and/or osteoconductive polymer fillings enables the implant to be multifunctional. Furthermore, its overall rate of incorporation into host bone may be more predictable based on the degradation rates of the polymer matrixes used.

As noted above, stimulatory factors, including osteoinductive and osteoconductive molecules, can be absorbed into, onto or coupled to the polymeric material and/or filler. Cells can also be seeded onto or into the graft or polymeric matrix. Alternatively, cells may be located within a bone progenitor tissue site of a patient in a clinical application, and the stimulatory factor composition applied to the site in order to effect, or promote, stimulatory factor transfer into bone progenitor cells *in vivo.* In transferring stimulatory factors into bone cells in humans, a preferred method involves first adding the stimulatory material to a bone-compatible matrix and then using the impregnated matrix to fill the perforations within the allograft in contact with an appropriate tissue site.

The following nonlimiting examples are provided to further illustrate the methods and materials generally disclosed herein.

### Example 1: Formation of Polymer-Allograft Composite.

### Materials and Methods

### Polymers

Three types of PLGA polymers were selected based on varying degradation rates: poly(d,l-lactic-co-glycolic acid) polymers composed of lactic acid and glycolic acid in the mole ratio of 50:50 (PLGA 50:50), in the mole ratio of 75:25 (PLGA 75:25), and in the mole ratio of 100:0 (PLA, poly (70-1-lactic-co-30-d,1-lactic acid)).

Lewis (1990) summarized degradation times for poly(lactide-co-glycolides) as follows in Table 1.

**TABLE 1:**

| **Polymer Degradation Rates** | |
|---|---|
| **Polymer** | **Approximate** Biodegradation Time, Months |
| Poly(L-lactide) | 18-24 |
| Poly(D,L-lactide) | 12-16 |
| Poly(glycolide) | 2-4 |
| Poly(D,L-lactide-co-glycolide) - 50:50 | 2 |
| Poly(d,L-lactide, co-glycolide) - 85:15 | 5 |

These degradation times represent values for defined geometries and masses. The three polymers proposed for this Phase I project represent a wide range of degradation rates and should nominally open the allograft pores at early (PLGA50:50), middle (PLGA75:25) and later (PLA) time points.

### Sample preparation

Cortical bone samples (12 mm × 5 mm × 5mm) were perforated with a pulsed Erbium:YAG laser (SEO Laser 1-2-3 Schwartz Electro Optics, Concord, MA) operating at a wavelength of 2940 nm. Holes of 610 µm diameter were drilled through the entire cortex. PLGA 50:50 and PLGA 75:25 were dissolved in glacial acetic acid to obtain a solution of concentration 50 mg/ml (5% w/v). The PLA was dissolved in glacial acetic acid to give a solution concentration of 35 mg/ml (3.5% w/v).

The polymer solution was forced into the holes using continuous evacuation and repressurization, the end point determination being the absence of bubbles from the laser holes. Approximately 1.5 µl of solution was forced into each hole. To remove the residual glacial acetic acid solvent, the polymer bone constructs were then frozen in an isopropanol-dry ice bath (-79° C).

The resulting frozen construct was then removed into a 300ml lyophilization flask and attached to a lyophilizer (Labconco, Freezedryer 8) operating at -40° C and a vacuum of less than 5 mm Hg for a period of 48 hours. This resulted in the formation of polymer foam plugs in the laser-drilled holes.

### In vitro methods

The PLGA 50:50, PLGA 75:25 and PLA foam bone constructs were placed in 20 ml tubes containing 10 ml of phosphate buffered saline (pH 7.4). The tubes were then left in a water bath operating at 60 cycles per minute and 37 °C. The constructs were evaluated at 1, 2 and 3 weeks. Surface scanning electron micrographs were taken at different time points to view the extent of degradation over time of the polymer from the laser drilled holes. The images were taken on an Amray 1000 SEM.

### Results

The observations from the scanning electron micrographs confirmed the varying degradation rates of the polymers. At time 0, the polymers completely filled the drilled holes. After one week of incubation the PLGA 50:50 (the most quickly degrading polymer) only partially filled the drilled hole. The PLGA 75:25 at the one-week time point showed slight degradation (as determined via surface roughening), but the polymer still substantially filled the hole. The PLA, on the other hand, showed minimal degradation; only a slight curvature was observed on the polymer surface. At the two-week time point, the PLGA 50:50 had completely degraded and the hole was essentially empty with only slight observed along the edge of the hole. At two weeks, the PLGA 75:25 showed partial degradation; the hole was partially filled with polymer. In comparison, the PLA still filled the hole with only slight degradation at the edge of the hole.

These results demonstrate that polymer-allograft composites were prepared, as demonstrated by adequate adherence of the polymer to the bone in the pores of the allograft.

## Claims

1. A method of producing a perforated and partially demineralized cortical bone allograft transplant, comprising filling the perforations in the perforated and partially demineralised cortical bone allograft with a porous matrix formed of a biodegradable synthetic polymer.

2. The method of claim 1 wherein the polymeric matrix further comprises material selected from the group consisting of bulking agents or filler, pH buffering material, osteoconductive molecules, and osteoinductive molecules.

3. The method of claim 1 wherein the polymer is a hydrolytically degradable synthetic polymer.

4. The method of claim 3 wherein the polymer is selected from the group consisting of polyhydroxyacids, polyanhydrides, polyacrylic acids, and polyalkanoates.

5. The method of claim 2 wherein the material is selected from the group consisting of hydroxyapatite, tricalcium phosphate, bioglass, ceramic materials and metal materials.

6. The method of claim 2 further comprising implanting cells involved in bone repair or growth with or adjacent to the matrix.

7. The method of claim 2 wherein the material is selected from the group consisting of bone morphogenic proteins, hormones, cytokines, growth factors, other molecules that regulate growth and differentiation of bone.

8. The method of claim 1 wherein the polymer is dissolved and placed in the perforations, then the solvent removed.

9. The method of claim 8 wherein the solvent is removed to leave a porous polymer structure.

10. These of a perforated and partially demineralized cortical bone allograft, wherein the perforations are filled with a porous matrix formed of a biodegradable synthetic polymer or the allograft is coated with a porous matrix, formed of a biodegradable synthetic polymer the matrix comprising material selected from the group consisting of bulking agents or filler, pH buffering material, osteoconductive molecules, and osteoinductive molecules in the manufacture of a medicament for delivery of molecules for enhancing bone repair or growth at a site in need of treatment thereof.

11. The use of claim 10 wherein the material is selected from the group consisting of bone morphogenic proteins, hormones, cytokines, growth factors, other molecules that regulate growth and differentiation of bone.

12. The use of claim 11 wherein the material is a gene encoding the material.

13. The use of claim 11 wherein the material is a protein.

14. The use of claim 10 wherein the material is provided in cells expressing the material.

15. The use of claim 14 wherein the cells are implanted at the site with the allograft or implant.

16. A perforated and partially demineralized cortical bone allograft or implant, wherein the perforations are filled or coated with a porous matrix formed of a biodegradable synthetic polymer.

17. The allograft or implant of claim 16 wherein the performations are filled with a polymeric matrix having a controlled rate of degradation.

18. The allograft or implant of claim 16 wherein the allograft or implant is coated with a polymeric matrix comprising material selected from the group consisting of bulking agents or filler, pH buffering material, osteoconductive molecules, and osteoinductive molecules.

19. The allograft or implant of claim 16 wherein the polymeric matrix further comprises material selected from the group consisting of bulking agents or filler, pH buffering material, osteoconductive molecules, and osteoinductive molecules.

20. The allograft or implant of claim 16 wherein the polymer is a hydrolytically degradable synthetic polymer.

21. The allograft or implant of claim 16 wherein the polymer is selected from the group consisting of polyhydroxyacids, polyanhydrides, polyacrylic acids, and polyalkanoates.

22. The allograft or implant of claim 16 wherein the polymeric matrix further comprises material selected from the group consisting of hydroxyapatite, tricalcium phosphate, bioglass, ceramic materials and metal materials.

23. The allograft or implant of claim 16 further comprising cells involved in bone repair or growth seeded onto or into the polymeric matrix.

24. The allograft or implant of claim 16 wherein the matrix further comprises material selected from the group consisting of bone morphogenic proteins, hormones, cytokines, growth factors, other molecules that regulate growth and differentiation of bone.

25. The use of a composition comprising an isolated osteotropic factor and a structural bone-compatible matrix in the manufacture of a medicament for stimulating bone progenitor cells located within a bone progenitor tissue site of a patient wherein the composition promotes expression of an osteotropic gene in the cells.

26. The use of claim 25, wherein the osteotropic gene is brought into contact with the structural bone-compatible matrix to form a matrix-gene composition and the matrix-gene composition is brought into contact with the tissue site.

27. The use of claim 25, wherein the osteotropic gene encodes a protein selected from the group consisting of parathyroid hormone, bone morphogenetic proteins, growth factors, growth factor receptors, cytokines, and chemotactic factors.

28. The use of claim 27, wherein the osteotropic gene encodes a protein selected from the group consisting of transforming growth factor, fibroblast growth factor, granulocyte/macrophage colony stimulating factor, epidermal growth factor, platelet derived growth factor, insulin-like growth factor, and leukemia inhibitory factor.

29. The use of claim 27, wherein the osteotropic protein is selected from the group consisting of TGF-alpha, TGF-beta, PTH, BMP-2, BMP-2A, BMP-2B, BMP-3, BMP-4, BMP-5, BMP-6, BMP-7 and BMP-8.

30. An allograft or implant according to any one of claims 16 to 24 for use in medicine.

## Patentansprüche

1. Verfahren zur Herstellung eines Allotranspiantats aus einem perforierten und teilweise entmineralisierten kortikalen Knochen, wobei das Verfahren das Füllen der Perforationen im Allotransplantat aus einem perforierten und teilweise entmineralisierten kortikalen Knochen mit einer porösen Matrix aus einem biologisch abbaubaren, synthetischen Polymer umfasst.

2. Verfahren nach Anspruch 1, wobei die polymere Matrix ferner ein Material umfasst, das aus der Gruppe ausgewählt ist, die aus Füllstoffen oder einem Streckmittel, einem Material zur Pufferung des pH, osteokonduktiven Molekülen und osteoinduktiven Molekülen besteht.

3. Verfahren nach Anspruch 1, wobei das Polymer ein hydrolytisch abbaubares, synthetisches Polymer ist.

4. Verfahren nach Anspruch 3, wobei das Polymer aus der Gruppe ausgewählt ist, die aus Polyhydroxysäuren, Polyanhydriden, Polyacrylsäuren und Polyalkanoaten besteht.

5. Verfahren nach Anspruch 2, wobei das Material aus der Gruppe ausgewählt ist, die aus Hydroxylapatit, Tricalciumphosphat, Bioglas, Keramikmaterialien und Metallmaterialien besteht.

6. Verfahren nach Anspruch 2, das ferner das Implantieren von Zellen, die an der Reparatur oder dem Wachstum von Knochen beteiligt sind, zusammen mit der oder angrenzend an die Matrix umfasst.

7. Verfahren nach Anspruch 2, wobei das Material aus der Gruppe ausgewählt ist, die aus *Bone Morphogenetic Proteins*, Hormonen, Cytokinen, Wachstumsfaktoren und anderen Molekülen, die das Wachstum und die Differenzierung von Knochen regulieren, besteht.

8. Verfahren nach Anspruch 1, wobei das Polymer gelöst und in die Perforationen gegeben wird und dann das Lösemittel entfernt wird.

9. Verfahren nach Anspruch 8, wobei das Lösemittel unter Zurücklassung einer porösen Polymerstruktur entfernt wird.

10. Verwendung eines Allotransplantats aus einem perforierten und teilweise entmineralisierten kortikalen Knochen, wobei die Perforationen mit einer porösen Matrix aus einem biologisch abbaubaren, synthetischen Polymer gefüllt sind oder das Allotransplantat mit einer porösen Matrix aus einem biologisch abbaubaren, synthetischen Polymer beschichtet ist, wobei die Matrix ein Material umfasst, das aus der Gruppe ausgewählt ist, die aus Füllstoffen oder einem Streckmittel, einem Material zur Pufferung des pH, osteokonduktiven Molekülen und osteoinduktiven Molekülen besteht, bei der Herstellung eines Arzneimittels für die Zufuhr von Molekülen zur Verstärkung der Reparatur oder des Wachstums von Knochen.

11. Verwendung nach Anspruch 10, wobei das Material aus der Gruppe ausgewählt ist, die aus *Bone Morphogenetic Proteins,* Hormonen, Cytokinen, Wachstumsfaktoren und anderen Molekülen, die das Wachstum und die Differenzierung von Knochen regulieren, besteht.

12. Verwendung nach Anspruch 11, wobei das Material ein Gen ist, das für das Material codiert

13. Verwendung nach Anspruch 11, wobei das Material ein Protein ist.

14. Verwendung nach Anspruch 10, wobei das Material in Zellen bereit gestellt wird, die das Material exprimieren.

15. Verwendung nach Anspruch 14, wobei die Zellen an der Stelle mit dem Allotransplantat oder Implantat implantiert werden.

16. Allotransplantat oder implantat aus einem perforierten und teilweise entmineralisierten kortikalen Knochen, wobei die Perforationen mit einer porösen Matrix aus einem biologisch abbaubaren, synthetischen Polymer gefüllt oder beschichtet sind.

17. Allotransplantat oder Implantat nach Anspruch 16, wobei die Perforationen mit einer polymeren Matrix, die mit kontrollierter Geschwindigkeit abgebaut wird, gefüllt sind.

18. Allotransplantat oder Implantat nach Anspruch 16, wobei das Allotransplantat oder Implantat mit einer polymeren Matrix beschichtet ist, die ein Material umfasst, das aus der Gruppe ausgewählt ist, die aus Füllstoffen oder einem Streckmittel, einem Material zur Pufferung des pH, osteokonduktiven Molekülen und osteoinduktiven Molekülen besteht.

19. Allotransplantat oder Implantat nach Anspruch 16, wobei die polymere Matrix femer ein Material umfasst, das aus der Gruppe ausgewählt ist, die aus Füllstoffen oder einem Streckmittel, einem Material zur Pufferung des pH, osteokonduktiven Molekülen und osteoinduktiven Molekülen besteht.

20. Allotransplantat oder Implantat nach Anspruch 16, wobei das Polymer ein hydrolytisch abbaubares, synthetisches Polymer ist.

21. Allotransplantat oder Implantat nach Anspruch 16, wobei das Polymer aus der Gruppe ausgewählt ist, die aus Polyhydroxysäuren, Polyanhydriden, Polyacrylsäuren und Polyalkanoaten besteht.

22. Allotransplantat oder Implantat nach Anspruch 16, wobei die polymere Matrix ferner ein Material umfasst, das aus der Gruppe ausgewählt ist, die aus Hydroxylapatit, Tricalciumphosphat, Bioglas, Keramikmaterialien und Metallmaterialien besteht

23. Allotransplantat oder implantat nach Anspruch 16, das ferner an der Reparatur oder dem Wachstum von Knochen beteiligte Zellen umfasst, die auf oder in die polymere Matrix ausgesät wurden.

24. Allotransplantat oder Implantat nach Anspruch 16, wobei die Matrix ferner ein Material umfasst, das aus der Gruppe ausgewählt ist, die aus Bone *Morphogenetic Proteins*, Hormonen, Cytokinen, Wachstumsfaktoren und anderen Molekülen, die das Wachstum und die Differenzierung von Knochen regulieren, besteht

25. Verwendung einer Zusammensetzung, die einen isolierten osteotropen Faktor und eine stützende, knochenkompatible Matrix umfasst, bei der Herstellung eines Arzneimittels für die Stimulierung von Knochenvorläuferzellen, die bei einem Patienten an einer Stelle mit Knochenvorläufergewebe lokalisiert sind, wobei die Zusammensetzung die Expression eines osteotropen Gens in den Zellen anregt.

26. Verwendung nach Anspruch 25, wobei das osteotrope Gen mit der stützenden, knochenkompatiblen Matrix in Kontakt gebracht wird, wodurch eine Zusammensetzung aus der Matrix und dem Gen gebildet wird, und wobei die Zusammensetzung aus der Matrix und dem Gen mit der Gewebestelle in Kontakt gebracht wird.

27. Verwendung nach Anspruch 25, wobei das osteotrope Gen für ein Protein codiert, das aus der Gruppe ausgewählt ist, die aus dem Parathormon, *Bone Morphogenetic Proteins*, Wachstumsfaktoren, Rezeptoren für Wachstumsfaktoren, Cytokinen und chemotaktischen Faktoren besteht.

28. Verwendung nach Anspruch 27, wobei das osteotrope Gen für ein Protein codiert, das aus der Gruppe ausgewählt ist, die aus dem *Transforming Growth Factor*, dem *Fibroblast Growth Factor*, dem *Granulocyte*/*Macrophage Colony Stimulating Factor*, dem *Epidermal Growth Factor,* dem *Platelet Derived Growth Factor*, dem *Insulin-like Growth Factor* und dem *Leukemia Inhibitory Factor* besteht

29. Verwendung nach Anspruch 27, wobei das osteotrope Protein aus der Gruppe ausgewählt ist, die aus TGF-alpha, TGF-beta, PTH, BMP-2, BMP-2A, BMP-2B, BMP-3, BMP-4, BMP-5, BMP-6, BMP-7 und BMP-8 besteht.

30. Allotransplantat oder Implantat nach einem beliebigen der Ansprüche 16 bis 24 für den Einsatz in der Medizin.

## Revendications

1. Procédé pour produire un transplant d'allogreffe de périoste perforé et partiellement déminéralisé, consistant à remplir les perforations dans l'allogreffe de périoste perforé et partiellement déminéralisé avec une matrice poreuse formée d'un polymère synthétique biodégradable.

2. Procédé selon la revendication 1, dans lequel la matrice polymère comprend en outre une matière choisie dans le groupe constitué des agents de volume ou d'une charge, de matière tampon pH, des molécules ostéoconductrices et des molécules ostéoinductives.

3. Procédé selon la revendication 1, dans lequel le polymère est un polymère synthétique hydrolytiquement dégradable.

4. Procédé selon la revendication 3, dans lequel le polymère est choisi dans le groupe constitué des polyhydroxyacides, des polyanhydrides, des acides polyacryliques et des polyalcanoates.

5. Procédé selon la revendication 2, dans lequel la matière est choisie dans le groupe constitué de l'hydroxyapatite, du phosphate tricalcique, du bioverre, des matières céramiques et des matières métalliques.

6. Procédé selon la revendication 2, consistant de plus à implanter des cellules impliquées dans la réparation ou la croissance osseuse avec la matrice ou de façon adjacente à celle-ci.

7. Procédé selon la revendication 2, dans lequel la matière est choisie dans le groupe constitué des protéines morphogènes de l'os, des hormones, des cytokines, des facteurs de croissance, d'autres molécules qui régulent la croissance et la différentiation de l'os.

8. Procédé selon la revendication 1, dans lequel le polymère est dissous et placé dans les perforations, puis le solvant est éliminé.

9. Procédé selon la revendication 8, dans lequel le solvant est éliminé pour laisser une structure polymère poreuse.

10. Utilisation d'une allogreffe de périoste perforé et partiellement déminéralisé, dans laquelle les perforations sont remplies d'une matrice poreuse formée d'un polymère synthétique biodégradable ou bien l'allogreffe est revêtu d'une matrice poreuse formée d'un polymère synthétique biodégradable, la matrice comprenant une matière choisie dans le groupe constitué des agents de volume ou d'une charge, de matière tampon pH, des molécules ostéoconductrices et des molécules ostéoinductives, dans la fabrication d'un médicament délivrant des molécules en vue de favoriser la réparation ou la croissance osseuse à un endroit nécessitant un traitement.

11. Utilisation selon la revendication 10, dans laquelle la matière est choisie dans le groupe constitué des protéines morphogènes de l'os, des hormones, des cytokines, des facteurs de croissance, d'autres molécules qui régulent la croissance et la différentiation de l'os.

12. Utilisation selon la revendication 11, dans laquelle la matière est un gène codant pour la matière.

13. Utilisation selon la revendication 11, dans laquelle la matière est une protéine.

14. Utilisation selon la revendication 10, dans laquelle la matière est prévue dans des cellules exprimant la matière.

15. Utilisation selon la revendication 14, dans laquelle les cellules sont implantées sur le site avec l'allogreffe ou l'implant.

16. Allogreffe ou implant de périoste perforé ou partiellement déminéralisé, dans lequel les perforations sont remplies ou revêtues d'une matrice poreuse, formée d'un polymère synthétique biodégradable.

17. Allogreffe ou implant selon la revendication 16, dans lequel les perforations sont remplies d'une matière polymère ayant une vitesse régulée de dégradation.

18. Allogreffe ou implant selon la revendication 16, dans lequel l'allogreffe ou l'implant est revêtu d'une matrice polymère comprenant une matière choisie dans le groupe constitué des agents de volume ou d'une charge, de matière tampon pH, des molécules ostéoconductrices et des molécules ostéoinductives.

19. Allogreffe ou implant selon la revendication 16, dans lequel la matrice polymère comprend en outre une matière choisie dans le groupe constitué des agents de volume ou d'une charge, de matière tampon pH, des molécules ostéoconductrices et des molécules ostéoinductives.

20. Allogreffe ou implant selon la revendication 16, dans lequel le polymère est un polymère synthétique hydrolytiquement dégradable.

21. Allogreffe ou implant selon la revendication 16, dans lequel le polymère est choisi dans le groupe groupe constitué des polyhydroxyacides, des polyanhydrides, des acides polyacryliques et des polyalcanoates.

22. Allogreffe ou implant selon la revendication 16, dans lequel la matrice polymère comprend de plus une matière choisie dans le groupe constitué de l'hydroxyapatite, du phosphate tricalcique, du bioverre, des matières céramiques et des matières métalliques.

23. Allogreffe ou implant selon la revendication 16, comprenant de plus des cellules impliquées dans la réparation ou la croissance osseuse inoculées sur ou dans la matrice polymère.

24. Allogreffe ou implant selon la revendication 16, dans lequel la matrice comprend en outre une matière choisie dans le groupe constitué des protéines morphogènes de l'os, des hormones, des cytokines, des facteurs de croissance, d'autres molécules qui régulent la croissance et la différentiation de l'os.

25. Utilisation d'une composition comprenant un facteur ostéotrope isolé et une matrice compatible à l'os structurelle dans la fabrication d'un médicament pour stimuler les cellules souches d'os situées à l'intérieur d'un site de tissu souche d'os d'un patient chez lequel la composition active l'expression d'un gène ostéotrope dans les cellules.

26. Utilisation selon la revendication 25, dans laquelle le gène ostéotrope est amené en contact avec la matrice compatible à l'os structurelle pour former une composition matrice-gène et la composition matrice-gène est amenée en contact avec le site du tissu.

27. Utilisation selon la revendication 25, dans laquelle le gène ostéotrope code pour une protéine choisie dans le groupe formé par l'hormone parathyroïdienne, les protéines morphogénétiques de l'os, les facteurs de croissance, les récepteurs de facteur de croissance, les cytokines et les facteurs chimiostatiques.

28. Utilisation selon la revendication 27, dans laquelle le gène ostéotrope code pour une protéine choisie dans le groupe formé par la transformation du facteur de croissance, le facteur de croissance de fibroblastes, le facteur stimulant les granulocytes et les macrophages, le facteur de croissance de l'épiderme, le facteur de croissance insulinomimétique et le facteur d'inhibition de la leucémie.

29. Utilisation selon la revendication 27, dans laquelle la protéine ostéotrope est choisie dans le groupe constitué de TGF-alpha, TGF-bêta, PTH, BMP-2, BMP-2A, BMP-2B, BMP-3, BMP-4, BMP-5, BMP-6, BMP-7 et BMP-8.

30. Allogreffe ou implant selon l'une quelconque des revendications 16 à 24, pour une utilisation en médecine.
